# EUROPEAN PATENT APPLICATION

(11) **EP 4 714 404 A1**
(43) Date of publication of application: **25.03.2026**
(21) Application number: 24818813.8
(22) Date of filing: 07.06.2024
(51) Int. Cl.: A61F 5/442

(54) **NURSING MACHINE AND CLEANING ASSEMBLY**

(30) Priority: 07.06.2023 CN 202321444227 U
(71) Applicant: Suzhou Alton Electrical & Mechanical Industry Co., Ltd., Suzhou, Jiangsu 215211 (CN)
(72) Inventor: LU, Weidong, Chicago Illinois (US); SONG, Qiang, Suzhou, Jiangsu 215211 (CN); LI, Wei, Suzhou, Jiangsu 215211 (CN)
(74) Representative: Ricker, Mathias
(86) International application number: PCT/CN2024/098284
(87) International publication number: WO 2024/251287

(57) **Abstract**

A nursing machine and a cleaning assembly (6) thereof. An excreta collection device (5) of the nursing machine is wearable on a body of a user to receive excreta of the user. The excreta collection device (5) includes an excreta recess (520) configured to receive the excreta of the user and a first spray nozzle (560) configured to spray water. When in butt joint with the excreta collection device (5), the cleaning assembly (6) covers the excreta recess (520) and the first spray nozzle (560), and an inner chamber (90) is formed between the cleaning assembly (6) and the excreta collection device (5), and the excreta recess (520) forms part of the inner chamber (90). The first spray nozzle (560) is capable of spraying water into the inner chamber (90).

## Description

The present disclosure claims the priority to Chinese Patent Application No. 202321444227.6, filed with the Chinese Patent Office on June 7, 2023, which is incorporated herein by reference in its entirety.

### Technical Field

The present disclosure relates to a cleaning assembly and a nursing machine with same.

### Background

A nursing machine, for sucking and clearing excreta of a user, generally includes a main machine, an excreta collection device, a briefs device, an excreta suction pipeline and a water supply pipeline that are connected between the main machine and the excreta collection device, etc. The main machine is internally provided with a water pump, a clean water container for water supply, and an excreta container for accommodating excreta. The excreta collection device is provided with an excreta recess for accommodating the excreta. The main machine sucks the excreta in the excreta recess into the excreta container via the excreta suction pipeline under the suction of a negative pressure source. The water pump supplies water in the clean water container to the excreta collection device via the water supply pipeline and sprays out the water via a spray nozzle of the excreta collection device to flush a lower body of the user and the excreta recess, or flush away the excreta. The sewage formed after the flushing water is mixed with the excreta in the excreta recess is sucked into the excreta container via the excreta suction pipeline under suction of the negative pressure source. The interior of the excreta suction pipeline, the interior of the excreta recess, etc. are polluted by the excreta, the sewage, etc., and thus the excreta suction pipeline, the excreta recess, etc. need to be sterilized and disinfected regularly. According to an existing disinfection method, it is common practice to immerse the excreta suction pipeline, the excreta collection device, etc. in a disinfectant solution for immersed sterilization and disinfection, which is troublesome to operate and needs to be improved.

### Summary

The present disclosure provides a nursing machine. When in butt joint with an excreta collection device, a cleaning assembly of the nursing machine covers an excreta recess, etc. of the excreta collection device, and an inner chamber is formed between the cleaning assembly and the excreta collection device. The excreta recess of the excreta collection device forms a part of the inner chamber, and water may be injected into the inner chamber by means of a spray nozzle of the excreta collection device, so that water injection is convenient. According to the design, the excreta recess may be effectively immersed by injecting water into the inner chamber. If the water in the inner chamber contains a disinfectant material, a disinfectant solution is formed. The inner chamber itself may effectively and conveniently accommodate sufficient water or disinfectant solution, so that the excreta recess may be effectively immersed in the accommodated water or disinfectant solution. Based on the design, the immersed disinfection may be conveniently performed on the excreta recess, etc. of the excreta collection device.

A nursing machine is provided. The nursing machine includes an excreta collection device, where the excreta collection device is wearable on a body of a user to receive excreta of the user, and the excreta collection device includes an excreta recess configured to receive the excreta of the user, and a first spray nozzle configured to spray water; the nursing machine further includes a cleaning assembly, where when in butt joint with the excreta collection device, the cleaning assembly covers the excreta recess and the first spray nozzle; when the cleaning assembly is in butt joint with the excreta collection device, an inner chamber is formed between the cleaning assembly and the excreta collection device, and the excreta recess forms a part of the inner chamber; and the first spray nozzle is capable of spraying water into the inner chamber.

In some embodiments, the cleaning assembly includes a concave space, when the cleaning assembly is in butt joint with the excreta collection device, the concave space is combined with the excreta recess, and the concave space forms a part of the inner chamber.

In some embodiments, the nursing machine further includes a briefs device wearable on the body of the user, where the briefs device includes a connection bracket and a crotch through opening, at least a part of the crotch through opening is delimited by the connection bracket, after the briefs device is worn on the body of the user, the excreta collection device is capable of indirectly worn on the body of the user by being in butt joint with the connection bracket, and the excreta collection device is configured for being optionally in butt joint with either one of the cleaning assembly and the connection bracket of the briefs device.

In some embodiments, when the cleaning assembly is in butt joint with the excreta collection device, the first spray nozzle is exposed to an interior of the inner chamber, and the first spray nozzle is capable of spraying water into the inner chamber to fill the inner chamber with water.

In some embodiments, the excreta collection device further includes an excreta discharge pipeline, where a front end of the excreta discharge pipeline is located at a rear end of the excreta recess, and directly communicates with the excreta recess; the nursing machine further includes a main machine and an excreta suction pipeline, where the excreta discharge pipeline forms a part of the excreta suction pipeline; the excreta suction pipeline further includes a first excreta suction hose device, where one end of the first excreta suction hose device is in butt joint with the excreta collection device, and an opposite end of the first excreta suction hose device is in butt joint with the main machine; the inner chamber, the excreta discharge pipeline, and the first excreta suction hose device are in fluid communication in sequence; the first spray nozzle supplies water to the excreta suction pipeline via the inner chamber, and the first spray nozzle is capable of spraying water into the inner chamber to fill the inner chamber, the excreta discharge pipeline, and the first excreta suction hose device with water; a first water conveying pipeline is configured to supply water to the first spray nozzle; and the main machine includes a water pump, where the water pump drives water to be conveyed via the first water conveying pipeline and sprayed out via the first spray nozzle.

In some embodiments, the nursing machine further includes a main machine and an excreta suction pipeline; the excreta collection device further includes an excreta discharge pipeline, where the excreta discharge pipeline forms a part of the excreta suction pipeline, the excreta suction pipeline further includes a first excreta suction hose device, and the first excreta suction hose device is configured for fluid communication between the excreta collection device and the main machine; the main machine includes a negative pressure source providing power for sucking excreta, and an excreta container, where under the suction of the negative pressure source, the excreta in the excreta recess are sucked into the excreta container via the excreta suction pipeline; the inner chamber, the excreta discharge pipeline, and the first excreta suction hose device are in fluid communication in sequence, and the first spray nozzle supplies water to the excreta suction pipeline via the inner chamber; the cleaning assembly further includes an air intake check valve, where the air intake check valve is configured for unidirectional air intake into the inner chamber, and after the air intake check valve is opened, air in an external environment enters the inner chamber via the air intake check valve; and under the suction of the negative pressure source, the air intake check valve is opened, and the air in the external environment enters the inner chamber via the air intake check valve.

The present disclosure further provides a cleaning assembly for a nursing machine. The nursing machine includes an excreta collection device, where the excreta collection device is wearable on a body of a user to receive excreta of the user; the excreta collection device includes an excreta recess configured to receive the excreta of the user, and a first spray nozzle configured to spray water; when in butt joint with the excreta collection device, the cleaning assembly covers the excreta recess and the first spray nozzle; and when the cleaning assembly is in butt joint with the excreta collection device, an inner chamber is formed between the cleaning assembly and the excreta collection device, the excreta recess forms a part of the inner chamber, and the first spray nozzle is capable of spraying water into the inner chamber.

### Brief Description of the Drawings

Fig. 1 is an exploded view of an excreta collection device and a briefs device of a nursing machine according to the present disclosure;
Fig. 2 is a perspective view of a briefs device of a nursing machine according to the present disclosure;
Fig. 3 is a perspective view of an excreta collection device, a briefs device, and all pipelines of a nursing machine after assembly according to the present disclosure;
Fig. 4 is an exploded view of an excreta collection device of a nursing machine according to the present disclosure;
Fig. 5 is a horizontal sectional view of a connection bracket and an excreta collection device that are in butt joint of a nursing machine according to the present disclosure;
Fig. 6 is a perspective view of a connection bracket of a briefs device of a nursing machine according to the present disclosure;
Fig. 7 is an exploded view of a connection bracket of a briefs device of a nursing machine according to the present disclosure;
Fig. 8 is a vertical sectional view of a cleaning assembly and an excreta collection device that are in butt joint according to the present disclosure;
Fig. 9 is a perspective view of a cleaning assembly and an excreta collection device that are in butt joint according to the present disclosure;
Fig. 10 is another perspective view of a cleaning assembly and an excreta collection device that are in butt joint according to the present disclosure;
Fig. 11 is a perspective view of a main machine (connected to all pipelines) of a nursing machine according to the present disclosure;
Fig. 12 is an enlarged view of a portion in Fig. 8;
Fig. 13 is an exploded view of an air intake check valve of a cleaning assembly according to the present disclosure;
Fig. 14 is a perspective view of a cleaning assembly according to the present disclosure;
Fig. 15 is another perspective view of a cleaning assembly according to the present disclosure;
Fig. 16 is an exploded view of a cleaning assembly according to the present disclosure; and
Fig. 17 is a perspective view of a main machine (with a part of a housing of a main machine hidden) of a nursing machine according to the present disclosure.

### Detailed Description of the Embodiments

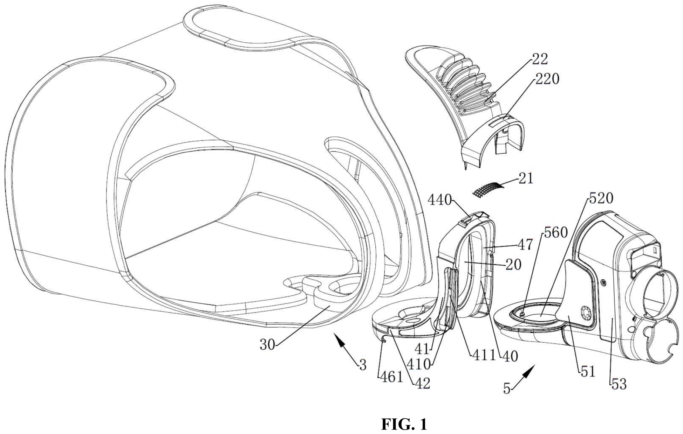

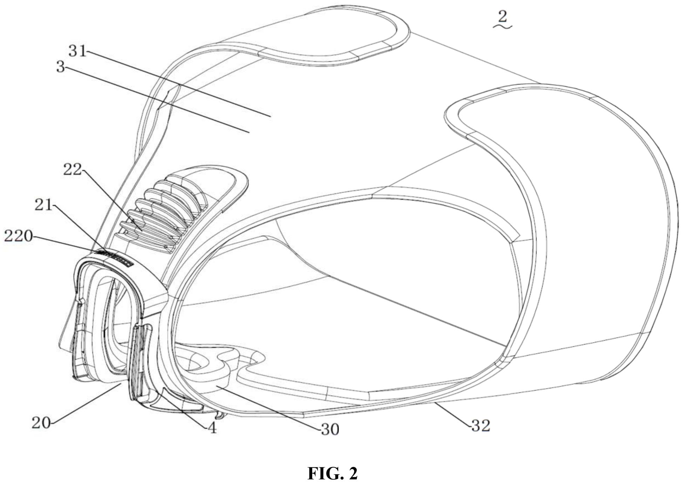

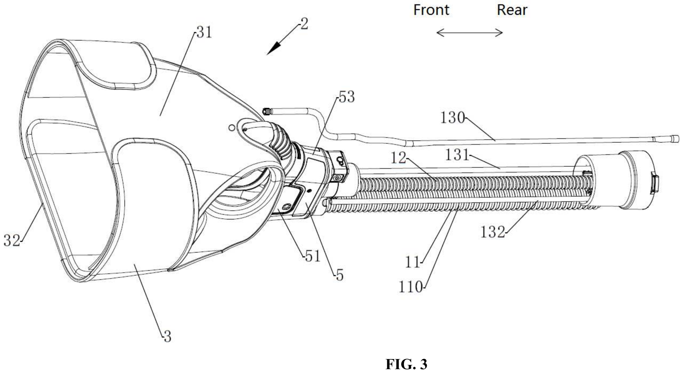

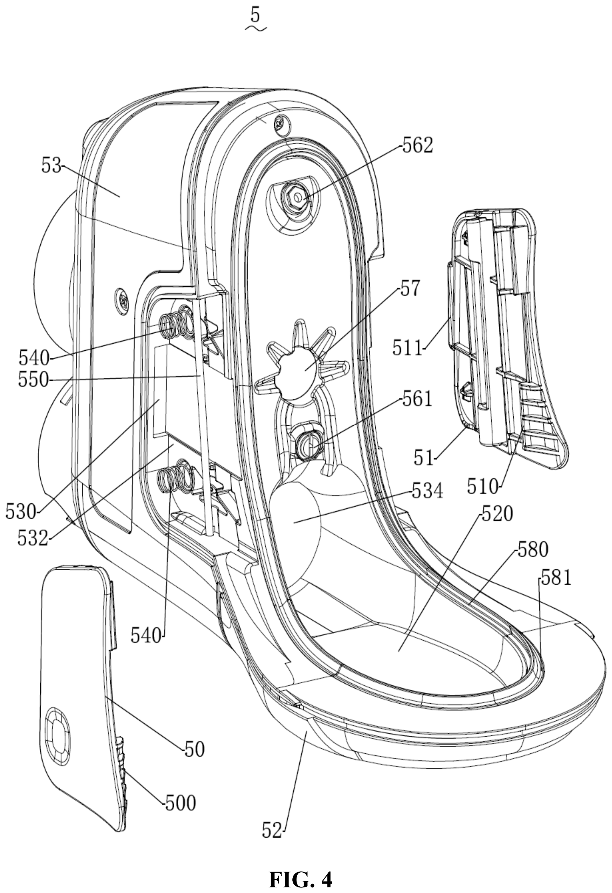

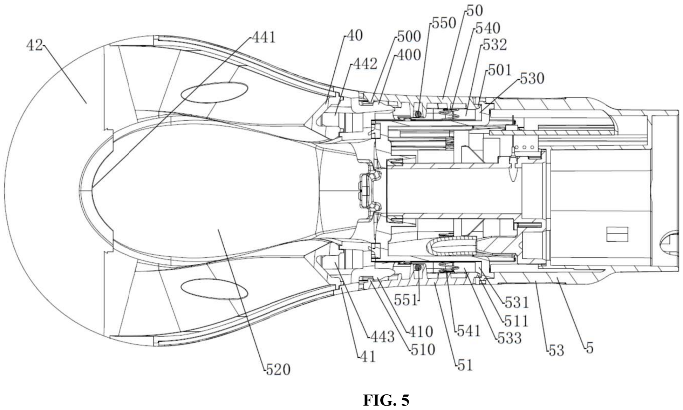

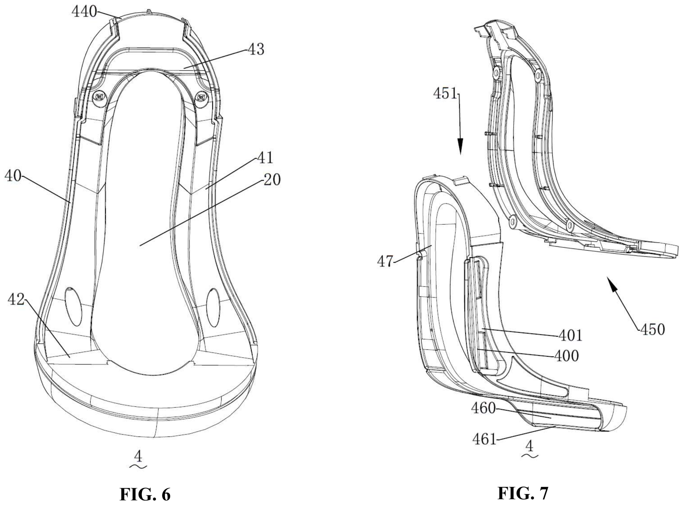

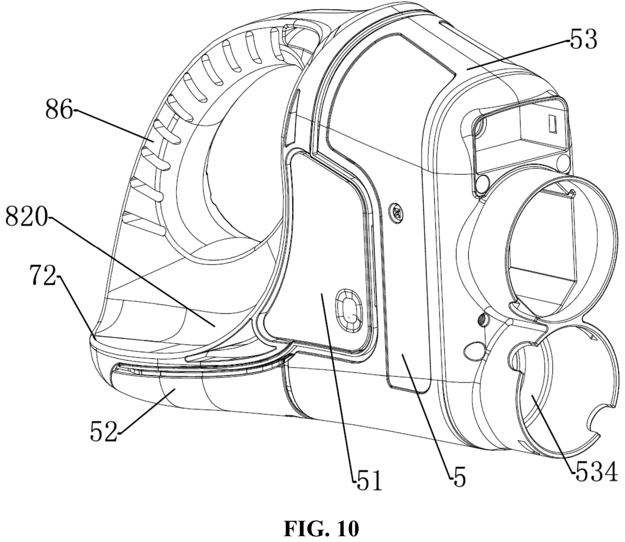

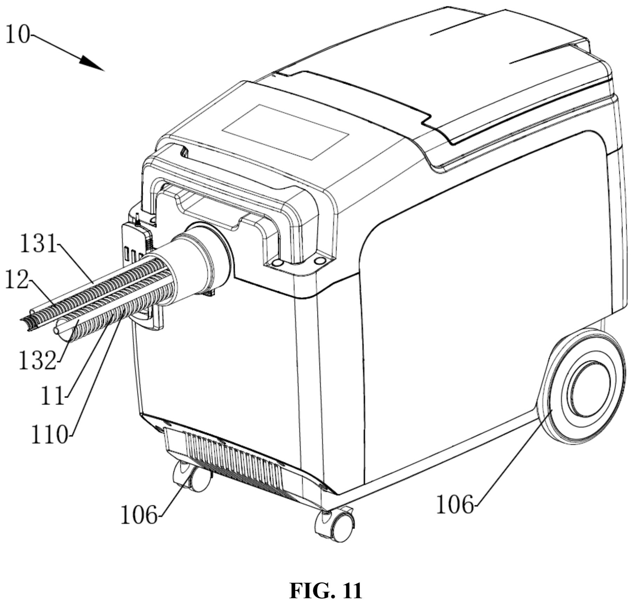

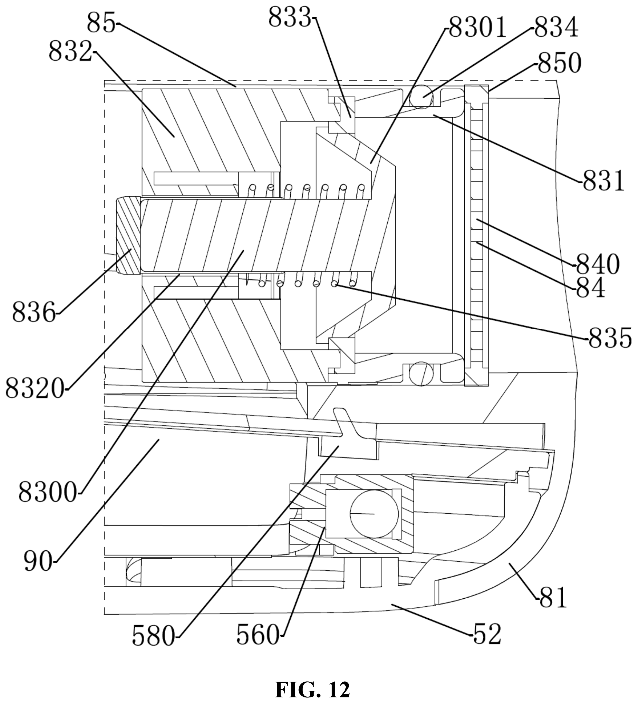

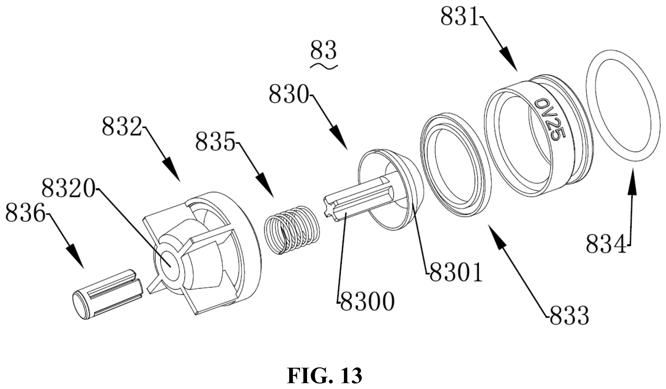

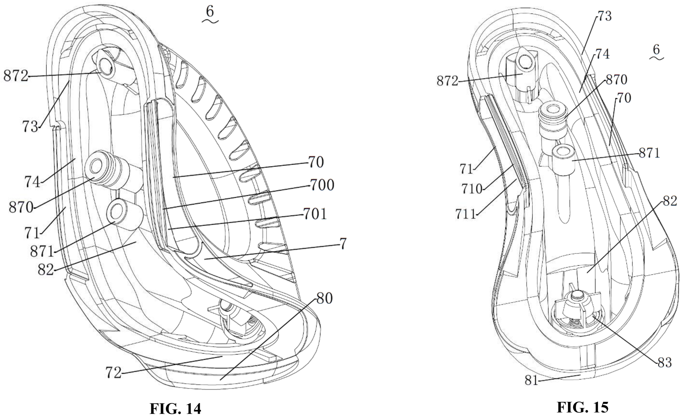

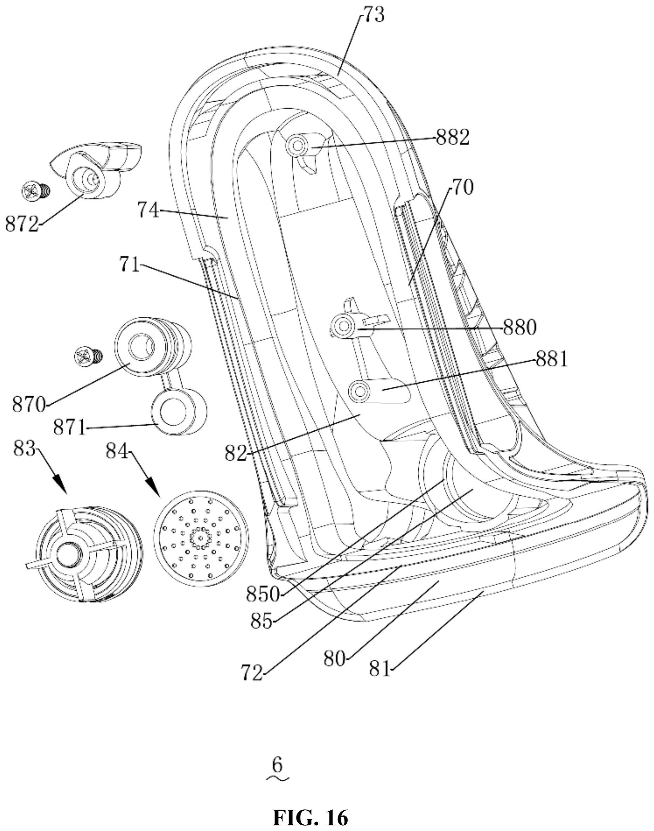

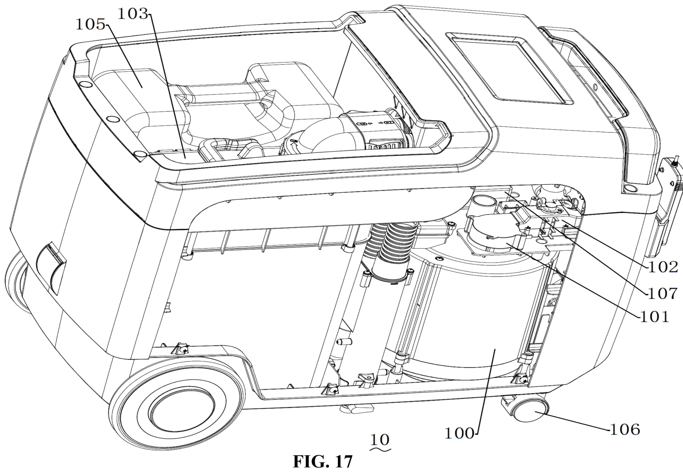

With reference to Figs. 1 to 17, a nursing machine of the present disclosure includes an excreta collection device 5 and a briefs device 2, where the excreta collection device 5 is wearable on a body of a user to receive excreta of the user; the briefs device 2 is wearable on the body of the user, and after the briefs device 2 is worn on the body of the user, the excreta collection device 5 may be indirectly worn on the body of the user by being in butt joint with the briefs device 2; the briefs device includes a briefs body 3, a connection bracket 4, and a crotch through opening 20, where the connection bracket 4 is connected to the briefs body 3, and the crotch through opening 20 is located at a crotch portion of the briefs device 2, and configured for excretion of the user; the excreta collection device 5 includes an excreta recess 520, where the excreta recess 520 is configured to receive the excreta of the user; the connection bracket 4 may be configured for butt joint between the briefs device 2 and the excreta collection device 5; the excreta collection device 5 is in butt joint with the connection bracket 4 detachably to be in butt joint with the briefs device 2; and the excreta collection device 5 may be indirectly worn on the body of the user by being in butt joint with the connection bracket 4. The briefs device 2 is in butt joint with the excreta collection device 5 rapidly and conveniently through the connection bracket 4. When in butt joint with the excreta collection device 5, the connection bracket 4 abuts on the excreta collection device 5.

With the deformation proneness, the briefs body 3 may be made of a flexible material. The briefs body 3 includes a leak-proof gasket 30, an upper coverage portion 31, and a lower coverage portion 32. The leak-proof gasket 30 abuts on the body of the user, and may be a gel gasket, a sponge gasket, an inflatable gasket, a latex gasket, etc. The leakage-proof gasket 30 is located at the crotch through opening 20 correspondingly, and may tightly abut against to the body of the user after the briefs device 2 is worn on the body of the user, to prevent the excreta of the user from leaking between the leakage-proof gasket 30 and the body of the user. When the briefs device 2 is worn on the body of the user, the upper coverage portion 31 correspondingly covers the abdomen of the user, and the lower coverage portion 32 correspondingly covers the buttocks and the back waist of the user.

The briefs device 2 further includes a urine cover 22, where the urine cover 22 is in a raised state, and is connected to the briefs body 3. Further, the urine cover 22 is bonded to the briefs body 3. The urine cover 22 may be made of a flexible material, such as silicone. A rear portion of the urine cover 22 is bonded to the connection bracket 4.

The connection bracket 4 is located at the crotch portion of the briefs device 2, and delimits at least a part of the crotch through opening 20. The connection bracket 4 includes a first left connection portion 40, a first right connection portion 41, a first U-shaped portion 42, and a second U-shaped portion 43, where the first left connection portion 40 is located at a left side of the crotch through opening 20, and the first right connection portion 41 is located at a right side of the crotch through opening 20. The first left connection portion 40 extends downwards from a left end of the second U-shaped portion 43, the first right connection portion 41 extends downwards from a right end of the second U-shaped portion 43, and the first left connection portion 40 and the first right connection portion 41 are attached to the briefs body 3 separately. Further, the first left connection portion 40 and the first right connection portion 41 are bonded to the briefs body 3 separately, to be firmly connected to the briefs body 3. When the briefs device 2 is worn on the body of the user, the first left connection portion 40 and the first right connection portion 41 are located between bases of two thighs of the user, and the first left connection portion 40 and the first right connection portion 41 are bilaterally symmetrical. The first U-shaped portion 42 is located at a front lower portion of the connection bracket 4, the second U-shaped portion 43 is located at a rear upper portion of the connection bracket 4, and the first left connection portion 40 and the first right connection portion 41 are connected between the first U-shaped portion 42 and the second U-shaped portion 43 separately. The first U-shaped portion 42, the first left connection portion 40, the second U-shaped portion 43, and the first right connection portion 41 are connected in sequence to delimit the crotch through opening 20. The second U-shaped portion 43 is connected to a rear portion of the urine cover 22. Further, the second U-shaped portion 43 is bonded to the rear portion of the urine cover 22.

The connection bracket 4 is provided with a first slot 460, where the first slot 460 is located in a front bottom side of the connection bracket 4, a front end of the excreta collection device 5 may be inserted into the first slot 460 forwards, and when the connection bracket 4 is butt joint with the excreta collection device 5, the front end of the excreta collection device 5 is embedded in the first slot 460. The connection bracket 4 further includes a first cambered stop wall 461, where the first cambered stop wall 461 is configured to delimit the first slot 460, and the first cambered stop wall 461 is connected to the first U-shaped portion 42, and located at a lower side of the first U-shaped portion 42. The first slot 460 is located at the lower side of the first U-shaped portion 42, and the first slot 460 is formed between the first U-shaped portion 42 and the first cambered stop wall 461. After inserted into the first slot 460, the front end of the excreta collection device 5 is attached to the first cambered stop wall 461.

The connection bracket 4 is provided with a first ventilation opening 440, an air outlet 441, and an airflow channel, where the air outlet 441 is configured to discharge air towards the excreta recess 520, the air outlet 441 is located in an edge of the crotch through opening 20, and the airflow channel enables communication between the first ventilation opening 440 and the air outlet 441. When the air pressure of the air outlet 441 is lower than that of the first ventilation opening 440, air may flow through the first ventilation opening 440, the airflow channel, and the air outlet 441 in sequence, so that the air outlet 441 is replenished with the air. The connection bracket 4 delimits the crotch through opening 20, the connection bracket 4 is annular, the first ventilation opening 440 is located in an outer periphery of the connection bracket 4, and the air outlet 441 is located in an inner periphery of the connection bracket 4. The first left connection portion 40, the first right connection portion 41, and the second U-shaped portion 43 are located at a rear side of the briefs body 3 separately. The first U-shaped portion 42 is located at a lower side of the briefs body 3, the air outlet 441 is formed in the first U-shaped portion 42, and the first ventilation opening 440 is formed in the second U-shaped portion 43.

The urine cover 22 is provided with a second ventilation opening 220, where the second ventilation opening 220 is located in a rear portion of the urine cover 22, and exposed to an exterior of the briefs device 2, and the first ventilation opening 440 is in fluid communication between the second ventilation opening 220 and the airflow channel. The second ventilation opening 220, the first ventilation opening 440, the airflow channel, and the air outlet 441 are in communication in sequence, so that an air conveying channel is formed. When the air pressure of the air outlet 441 is lower than that of the second ventilation opening 220, air outside the briefs device 2 may flow through the first ventilation opening 440, the airflow channel, and the air outlet 441 in sequence, so that the air outlet 441 is replenished with the air outside the briefs device 2.

The first ventilation opening 440 is formed in a top side of the second U-shaped portion 43, and the second ventilation opening 220 is located above the first ventilation opening 440, and opposite the first ventilation opening 440 in an up-down direction. The briefs device 2 further includes a protective net 21, where the protective net 21 is adjacent to the second ventilation opening 220 and the first ventilation opening 440, and the protective net 21 is located between the second ventilation opening 220 and the first ventilation opening 440. The protective net 21 is bonded to the rear portion of the urine cover 22. The protective net 21 is located at a lower side of the second ventilation opening 220, and covers the first ventilation opening 440. The protective net 21 takes a role of filtering and protecting, so that foreign matter carried by the airflow flowing through the second ventilation opening 220 are stopped by the protective net 21, , to prevented from entering the first ventilation opening 440.

The airflow channel includes a left channel 442 and a right channel 443, where the left channel 442 enables communication between the first ventilation opening 440 and the air outlet 441, and the right channel 443 enables communication between the first ventilation opening 440 and the air outlet 441. The first left connection portion 40 and the first right connection portion 41 are hollow, the left channel 442 penetrates the first left connection portion 40, and the right channel 443 penetrates the first right connection portion 41. An inner space of the first left connection portion 40 forms a part of the left channel 442, and an inner space of the first right connection portion 41 forms a part of the right channel 443. After entering the first ventilation opening 440 through the left channel 442 and the right channel 443, air is branched to flow forwards, and merges at the outlet 441. The connection bracket 4 is hollow to form the airflow channel inside the connection bracket 4. The connection bracket 4 includes a first frame body 450 and a second frame body 451, where the first frame body 450 and the second frame body 451 are combined together to form the connection bracket 4, and the airflow channel is formed between the first frame body 450 and the second frame body 451. The first frame body 450 and the second frame body 451 are assembled and buckled together from front to back, and locked together from front to back through a plurality of screws. The joint between the first frame body 450 and the second frame body 451 is sealed with glue to prevent the airflow in the airflow channel from leaking out through the joint.

The first left connection portion 40 includes a first left buckling portion 400, and the first right connection portion 41 includes a first right buckling portion 410, where the first left buckling portion 400 and the first right buckling portion 410 are located at a left side and a right side of the connection bracket 4 respectively, and the first left buckling portion 400 and the first right buckling portion 410 may be buckling slots and/or buckling protrusion separately.

The excreta collection device 5 may be in butt joint with the connection bracket 4 forwards to be connected to the briefs device 2. The excreta collection device 5 includes a locking mechanism, where the locking mechanism includes a left buckling member 50 and a right buckling member 51, and the left buckling member 50 and the right buckling member 51 are located at a left side and a right side of the excreta collection device 5 respectively. The left buckling member 50 may be buckled on the first left buckling portion 400, and the right buckling member 51 may be buckled on the first right buckling portion 410. The left buckling member 50 is provided with a left buckling protrusion 500, and the right buckling member 51 is provided with a right buckling protrusion 510. When the connection bracket 4 is in butt joint with the excreta collection device 5, the left buckling protrusion 500 snaps with the first left buckling portion 400, and the right buckling protrusion 510 snaps with the first right buckling portion 410. Along with the forward insertion of the front end of the excreta collection device 5 into the first slot 460, the left buckling member 50 snaps with the first left buckling portion 400 and the right buckling member 51 snaps with the first right buckling portion 410, so that the excreta collection device 5 and the connection bracket 4 are locked together.

When the connection bracket 4 is in butt joint with the excreta collection device 5, the first left connection portion 40 and the first right connection portion 41 are attached to the excreta collection device 5, and located between the excreta collection device 5 and the briefs body 3 separately. The excreta collection device 5 further includes a tray portion 52 and a body portion 53, where the excreta recess 520 is concavely arranged at the tray portion 52, and serves as a part of the tray portion 52. The left buckling member 50 and the right buckling member 51 are pivotally connected to the body portion 53. The left buckling member 50 and the right buckling member 51 are located at a left side and a right side of the body portion 53 respectively. When in butt joint with the excreta collection device 5, the connection bracket 4 abuts on the body portion 53 and the tray portion 52. When the connection bracket 4 is in butt joint with the excreta collection device 5, the first U-shaped portion 42 abuts on the tray portion 52, and the second U-shaped portion 43 abuts on the body portion 53. When the connection bracket 4 is in butt joint with the excreta collection device 5, the first left connection portion 40 and the first right connection portion 41 abut against the body portion 53. The tray portion 52 extends forwards from a lower portion of the body portion 53. A front end of the tray portion 52 forms the front end of the excreta collection device 5, so that the front end of the tray portion 52 may be inserted into the first slot 460 forwards.

The left buckling member 50 and the right buckling member 51 are rotatable, the excreta collection device 5 is further provided with a left elastic member 540 corresponding to the left buckling member 50, and a right elastic member 541 corresponding to the right buckling member 51. The left elastic member 540 is located between the body portion 53 and the left buckling member 50, and the right elastic member 541 is located between the body portion 53 and the right buckling member 51. When the connection bracket 4 is in butt joint with the excreta collection device 5, the left buckling member 50 keeps in a state of snapping with the first left buckling portion 400 under the action of an elastic force of the left elastic member 540, and the right buckling member 51 keeps in a state of snapping with the first right buckling portion 410 under the action of an elastic force of the right elastic member 541. The left elastic member 540 and the right elastic member 541 may be springs. Along with the forward insertion of the excreta collection device 5 into the connection bracket 4, the left buckling member 50 snaps with the first left buckling portion 400 through elastic pushing by the left elastic member 540 and the right buckling member 51 snaps with the first right buckling portion 410 through elastic pushing by the right elastic member 541. The left buckling protrusion 500 protrudes rightwards, and the right buckling protrusion 510 protrudes leftwards. **In** a process of inserting the excreta collection device 5 into the connection bracket 4 forwards, the left buckling protrusion 500 is buckled rightwards on the first left buckling portion 400 through elastic pushing of the left elastic member 540 on the left buckling member 50, and the right buckling protrusion 510 is buckled leftwards on the first right buckling portion 410 through elastic pushing of the right elastic member 541 on the right buckling member 51. Thus, the left buckling member 50 and the right buckling member 51 are basically synchronously and automatically buckled on the corresponding left buckling portion 400 and the right buckling portion 410 through elastic pushing by the left elastic member 540 and the right elastic member 541.

The excreta collection device 5 is further provided with a left rotary shaft 550 and a right rotary shaft 551, where the left rotary shaft 550 forms a rotary shaft of the left buckling member 50, and the right rotary shaft 551 forms a rotary shaft of the right bucking member 51. The left buckling member 50 is rotatably connected to the body portion 53 through the left rotary shaft 550, and the right buckling member 51 is rotatably connected to the body portion 53 through the right rotary shaft 551. The left rotary shaft 550 and the right rotary shaft 551 extend in an up-down direction separately. The left rotary shaft 550 and the left rotary shaft 550 may be made of metal (such as stainless steel). The left buckling protrusion 500 and the left elastic member 540 are located at a front side and a rear side of the left rotary shaft 550 respectively, the left buckling protrusion 500 is located in front of the left rotary shaft 550, and the left elastic member 540 is located behind the left rotary shaft 550. The right buckling protrusion 510 and the right elastic member 541 are located at a front side and a rear side of the right rotary shaft 551 respectively, the right buckling protrusion 510 is located in front of the right rotary shaft 551, and the right elastic member 541 is located behind the right rotary shaft 551.

A left limiting groove 530 and a right limiting groove 531 are formed in the body portion 53, a part of the left buckling member 50 extends into the left limiting groove 530, and a part of the right buckling member 51 extends into the right limiting groove 531. The part of the left buckling member 50 may abut against a left inner surface of the left limiting groove 530 leftwards through pushing of the left elastic member 540 on the left buckling member 50. The part of the right buckling member 51 may abut against a right inner surface of the right limiting groove 531 rightwards through pushing of the right elastic member 541 on the right buckling member 51. The left limiting groove 530 and the right limiting groove 531 are recesses dented backwards. In the example, the part of the left buckling member 50 is a first protrusion portion 501 protruding backwards and located at a rear end of the left buckling member 50, and the part of the right buckling member 51 is a second protrusion portion 511 protruding backwards and located at a rear end of the right buckling member 51.

The body portion 53 is further provided with a first left dented region 532 and a first right dented region 533, where the first left dented region 532 and the first right dented region 533 are located at the left side and the right side of the body portion 53 respectively. The left buckling member 50 is embedded in and covers the first left dented region 532, and the right buckling member 51 is embedded in and covers the first right dented region 533. The first left connection portion 40 is further provided with a second left dented region 401, the first right connection portion 41 is further provided with a second right dented region 411. The first left buckling portion 400 is arranged in the second left dented region 401, and the first right buckling portion 410 is arranged in the second right dented region 411. When the connection bracket 4 is in butt joint with the excreta collection device 5, a front portion of the left buckling member 50 is embedded in the second left dented region 401, and a front portion of the right buckling member 51 is embedded in the second right dented region 411. The second left dented region 401 and the second right dented region 411 are bilaterally symmetrical. The left elastic member 540 is located in the first left dented region 532, and the right elastic member 541 is located in the first right dented region 533.

When in use, the briefs device 2 is generally worn on the body of the user first, and then, the excreta collection device 5 is in butt joint with the connection bracket 4 forwards. The left buckling member 50 snaps with the first left buckling portion 400, and the right buckling member 51 snaps with the first right buckling portion 410, so that the briefs device 2 and the excreta collection device 5 are locked together. During unlocking, the rear portion of the left buckling member 50 and the rear portion of the right buckling member 51 are pressed in a direction close to each other with hands, so that the front portion of the left buckling member 50 tilts leftwards, and the front portion of the right buckling member 51 tilts rightwards. Thus, the left buckling protrusion 500 moves leftwards to be separated from the first left buckling portion 400 to release buckling. The right buckling protrusion 510 moves rightwards to be separated from the first right buckling portion 410 to release buckling. **In** this case, the excreta collection device 5 may be moved backwards to be separated from the briefs device 2 in the front-rear direction.

The excreta collection device 5 further includes a first spray nozzle 560 configured to spray water, a second spray nozzle 561 configured to spray water, a third spray nozzle 562 configured to spray water, and a drying air outlet 57, where the first spray nozzle 560 is arranged at the tray portion 52, and may be configured to flush away the excreta in the excreta recess 520. Further, the first spray nozzle 560 is located at a front side of the excreta recess 520, to spray water backwards to flush away the excreta in the excreta recess 520 backwards. The second spray nozzle 561 is arranged at the body portion 53, located at the front side of the body portion 53 and a rear upper side of the excreta recess 520, to spray water forwards to flush the anus of the user. The third spray nozzle 562 is arranged at the body portion 53, located at the front side of the body portion 53 and an upper side of the second spray nozzle 561, to flush the interior of the urine cover and/or a urination portion of the user. The drying air outlet 57 is located at the upper side of the second spray nozzle 561 and between the second spray nozzle 561 and the third spray nozzle 562, to dry a lower body of the user and facilitate drying of the excreta recess 520. The drying air outlet 57 may also be configured to replenish the vicinity of the excreta recess 520 with air, so that the excreta collected in the excreta recess 520 may be sucked away more sufficiently. When the connection bracket 4 is in butt joint with the excreta collection device 5, the air outlet 441 is adjacent to the upper side of the excreta collection device 520, and is located at an upper side of the front end of the excreta recess 520. Through such arrangement, the excreta recess 520 may be effectively replenished with air, and the excreta collected in the excreta recess 520 may be sucked backwards more sufficiently. When the connection bracket 4 is in butt joint with the excreta collection device 5, the air outlet 441 is adjacent to the first spray nozzle 560, and is located at the upper side of the first spray nozzle 560.

The excreta collection device 5 further includes a flexible sealing gasket 580 and a annular groove 581, where the flexible sealing gasket 580 is annular in shape corresponding to the annular groove 581, and the flexible sealing gasket 580 is arranged in the annular groove 581, and bonded to an inner surface of the annular groove 581. The annular groove 581 is provided in the body portion 53 and the tray portion 52, a part of the annular groove 581 is concavely arranged at the front side of the body portion 53, and the other part of the annular groove 581 is concavely arranged at the upper side of the tray portion 52. The flexible sealing gasket 580 surrounds an outer periphery of the excreta recess 520. When the connection bracket 4 is in butt joint with the excreta collection device 5, the flexible sealing gasket 580 is pressed between the excreta collection device 5 and the connection bracket 4 to seal between the excreta collection device 5 and the connection bracket 4 , to prevent the excreta in the excreta recess 520 from leaking out from between the excreta collection device 5 and the connection bracket 4. The flexible sealing gasket 580 is annular in shape corresponding to the connection bracket 4, and may be made of silicone. The connection bracket 4 is further provided with a first annular abutting surface 47 corresponding to the flexible sealing gasket 580, where the first annular abutting surface 47 is arranged on the first left connection portion 40, the first right connection portion 41, the first U-shaped portion 42, and the second U-shaped portion 43. When the connection bracket 4 is in butt joint with the excreta collection device 5, the flexible sealing gasket 580 is pressed against the first annular abutting surface 47 to realize sealing between the excreta collection device 5 and the connection bracket 4.

The excreta collection device 5 may be in butt joint with the connection bracket 4 forwards to be connected to the briefs device 2. After the excreta collection device 5 is in butt joint with the connection bracket 4 forwards, the briefs device 2 is located in front of the body portion 53. After the excreta collection device 5 is in butt joint with the connection bracket 4 forwards, the connection bracket 4 abuts on the body portion 53 and the tray portion 52. After the excreta collection device 5 is in butt joint with the connection bracket 4 forwards, the first left connection portion 40 and the first right connection portion 41 are located at the front side of the body portion 53, and abut against the body portion 53 separately. After the excreta collection device 5 is in butt joint with the connection bracket 4 forwards, the first U-shaped portion 42 is located at the upper side of the tray portion 52, and abuts on the tray portion 52. After the excreta collection device 5 is in butt joint with the connection bracket 4 forwards, the second U-shaped portion 43 is located at the front side of the body portion 53 and abuts on the body portion 53. When the connection bracket 4 is in butt joint with the excreta collection device 5, the urine cover 22 is located above the excreta recess 520, and the crotch through opening 20 is combined with the excreta recess 520, so that the excreta of the user enter the excreta recess 520 through the crotch through opening 20.

The nursing machine further includes a main machine 10, an excreta suction pipeline 11, an air conveying pipeline 12, a first water conveying pipeline 130, a second water conveying pipeline 131, and a third water conveying pipeline 132. The first water conveying pipeline 130 may be configured to supply water to the first spray nozzle 560, the second water conveying pipeline 131 may be configured to supply water to the second spray nozzle 561, and the third water conveying pipeline 132 may be configured to supply water to the third spray nozzle 562. The main machine 10 is provided with a negative pressure source 100 providing power for sucking the excreta, a fan 101, a heater 102, an excreta container 103, a second excreta suction hose device, a clean water container 105, a water pump 107, and wheels 106. The negative pressure source 100, the fan 101, the heater 102, the excreta container 103, and the water pump 107 are located inside the main machine 10. The water pump 107 is located downstream of the clean water container 105, to suck liquid from the clean water container 105. Water sprayed out from the first spray nozzle 560, the second spray nozzle 561, and the third spray nozzle 562 is sourced from the clean water container 105. All the water conveying pipelines 130, 131, and 132 are configured to be in fluid communication between the main machine 10 and the excreta collection device 5. The water pump 107 may drive water to be conveyed via the first water conveying pipeline 130, and sprayed out via the first spray nozzle 560. The water pump 107 may also drive water to be conveyed via the second water conveying pipeline 131, and sprayed out via the second spray nozzle 561. The water pump 107 may further drive water to be conveyed via the third water conveying pipeline 132, and sprayed out via the third spray nozzle 562. The wheels 106 are located at a bottom of the main machine 10, and configured for movement of the main machine 10.

The second excreta suction hose device is located inside the main machine 10, and serves as a part of the excreta suction pipeline 11. The excreta suction pipeline 11 is configured for fluid communication between the excreta collection device 5 and the main machine 10. Further, the excreta suction pipeline 11 is configured for fluid communication between the excreta recess 520 of the excreta collection device 5 and the excreta container 103 of the main machine 10. The negative pressure source 100 is provided with an electric motor and an impeller fixed to a rotary shaft of the electric motor. The impeller may rotate under driving by the rotary shaft of the electric motor to generate a suction force. The negative pressure source 100 is located downstream of the excreta container 103. Under the suction of the negative pressure source 100, the excreta in the excreta recess 520 are sucked into the excreta container 103 via the excreta suction pipeline 11. The excreta collection device 5 is further provided with an excreta discharge pipeline 534, where the excreta discharge pipeline 534 is located at a rear side of the excreta recess 520, and extends backwards from the excreta recess 520. The excreta discharge pipeline 534 is arranged at the body portion 53, and extends backwards from the excreta recess 520 to the rear side of the body portion 53. A front end of the excreta discharge pipeline 534 is located at the rear end of the excreta recess 520, and directly communicates with the excreta recess 520. The excreta discharge pipeline 534 forms a part of the excreta suction pipeline 11. The excreta suction pipeline 11 further includes a first excreta suction hose device 110, where the first excreta suction hose device 110 is located outside the main machine 10, and configured for fluid communication between the excreta collection device 5 and the main machine 10. One end of the first excreta suction hose device 110 is in butt joint with the excreta collection device 5, and an opposite end of the first excreta suction hose device 110 is in butt joint with the main machine 10. Further, the one end of the first excreta hose device 110 is in butt joint with a rear end of the excreta discharge pipeline 534, and the excreta recess 520, the excreta discharge pipeline 534, the first excreta suction hose device 110, the second excreta suction hose device, and the excreta container 103 may be in fluid communication in sequence. Under the suction of the negative pressure source 100, the excreta in the excreta recess 520 are sucked into the excreta container 103 via the excreta discharge pipeline 534, the first excreta suction hose device 110, and the second excreta suction hose device.

After the briefs device 2 is worn on the body of the user, and the excreta collection device 5 is in butt joint with the connection bracket 4, under the suction of the negative pressure source 100, the air pressure in the excreta recess 520 is lower than that of the external environment, external air flows through the second ventilation opening 220, the protective net 21, the first ventilation opening 440, the airflow channel, and the air outlet 441 in sequence to flow towards the excreta recess 520, so as to replenish the excreta recess 520 with the air. Thus, the excreta in the excreta recess 520 may be sucked more sufficiently. The drying air outlet 57 forms an air outlet of the air conveying pipeline 12, and the airflow driven by the fan 101 is conveyed to the excreta collection device 5 via the air conveying pipeline 12, and discharged via the drying air outlet 57, so as to dry the lower body of the user. A part of the air conveying pipeline 12 is located in the main machine 10, to butt with the heater 102. The airflow driven by the fan 101 is conveyed to the excreta collection device 5 via the air conveying pipeline 12 after passing through the heater 102. Thus, warm air is discharged through the drying air outlet 57 to dry the lower body of the user.

The nursing machine further includes the cleaning assembly 6, where the cleaning assembly 6 is in butt joint with the excreta collection device 5 detachably. The cleaning assembly 6 may be in butt joint with the excreta collection device 5 backwards, and the cleaning assembly 6 and the excreta collection device 5 may be referred to as a working head for the nursing machine. **In** other words, the cleaning assembly 6 and the excreta collection device 5 form the working head for the nursing machine. The excreta collection device 5 is in butt joint with the connection bracket 4 of the briefs device 2 to be in butt joint with the briefs device 2. The excreta collection device 5 may be in butt joint with either one of the briefs device 2 and the cleaning assembly 6. From another view, the excreta collection device 5 may be in butt joint with either one of the connection bracket 4 and the cleaning assembly 6. To be specific, the excreta collection device 5 may release the butt joint with the connection bracket 4 and may be in butt joint with the cleaning assembly 6, or may release the butt joint with the cleaning assembly 6 and may be in butt joint with the connection bracket 4. The excreta collection device 5 may be in butt joint with either one of the connection bracket 4 and the cleaning assembly 6. When the excreta collection device 5 is in butt joint with the connection bracket 4, the locking mechanism is buckled on the connection bracket 4. When the excreta collection device 5 is in butt joint with the cleaning assembly 6, the locking mechanism is buckled on the cleaning assembly 6. When the excreta collection device 5 is in butt joint with the connection bracket 4, the left buckling member 50 and the right buckling member 51 snap with the connection bracket 4. When the excreta collection device 5 is in butt joint with the cleaning assembly 6, the left buckling member 50 and the right buckling member 51 snap with the cleaning assembly 6.

The cleaning assembly 6 includes a frame opening portion 7, a concave space 82, an air intake check valve 83, a protective plate 84, a mounting hole 85, a handle 86, a first plug 870, a second plug 871, a third plug 872, and an outer wall 820. The frame opening portion 7 is configured for butt joint between the cleaning assembly 6 and the excreta collection device 5. The excreta collection device 5 may be in butt joint with the frame opening portion 7 detachably, the frame opening portion 7 is annular, the concave space 82 is formed by denting the frame opening portion 7 into the cleaning assembly 6, the frame opening portion 7 is annular, and extends along an edge of the concave space 82 to form a loop. The air intake check valve 83 is configured for unidirectional air intake into the concave space 82. The protective plate 84 is provided with a plurality of air intake holes, and located upstream of the air intake check valve 83. The porous protective plate 84 is located upstream of the air intake check valve 83 to take a role of filtering and protecting, so as to stop foreign matter , to prevent the air intake check valve 83 from being damaged by touching.

The frame opening portion 7 includes a second left connection portion 70, a second right connection portion 71, a third U-shaped portion 72, and a fourth U-shaped portion 73, where the second left connection portion 70 is located at a left side of the frame opening portion 7, the second right connection portion 71 is located at the right side of the frame opening portion 7, and the second left connection portion 70 and the second right connection portion 71 are located on a left side and a right side of the concave space 82 respectively. The second left connection portion 70 extends downwards from a left end of the fourth U-shaped portion 73, and the second right connection portion 71 extends downwards from a right end of the fourth U-shaped portion 73. The third U-shaped portion 72 is located at a front lower portion of the frame opening portion 7, the fourth U-shaped portion 73 is located at a rear upper portion of the frame opening portion 7, the second left connection portion 70 and the second right connection portion 71 are connected between the third U-shaped portion 72 and the fourth U-shaped portion 73 separately, and the third U-shaped portion 72, the second left connection portion 70, the fourth U-shaped portion 73, and the second right connection portion 71 are connected in sequence to form a loop.

The cleaning assembly 6 is further provided with a second slot 80 and a second cambered stop wall 81, where the second slot 80 is located in a front bottom side of the cleaning assembly 6, and the front end of the excreta collection device 5 may be inserted into the second slot 80 forwards. When the cleaning assembly 6 is in butt joint with the excreta collection device 5, the front end of the excreta collection device 5 is embedded in the second slot 80. The second cambered stop wall 81 is configured to delimit the second slot 80, connected to the third U-shaped portion 72, and located at a lower side of the third U-shaped portion 72. The second slot 80 is located at the lower side of the third U-shaped portion 72, and formed between the third U-shaped portion 72 and the second cambered stop wall 81. The front end of the excreta collection device 5 is attached to the second cambered stop wall 81 after inserted into the second slot 80. After the front end of the excreta collection device 5 is capable of inserting into the second slot 80, a bottom surface of the excreta collection device 5 is aligned with a bottom surface of the second cambered stop wall 81, and a front end of the tray portion 52 may be inserted into the second slot 80 forwards.

The second left connection portion 70 includes a second left buckling portion 700, the second right connection portion 71 includes a second right buckling portion 710, and the second left buckling portion 700 and the second right buckling portion 710 are located at the left side and the right side of the frame opening portion 7 respectively. The second left buckling portion 700 and the second right buckling portion 710 may be buckling protrusions separately. The excreta collection device 5 may be in butt joint with the cleaning assembly 6 forwards. To be specific, the cleaning assembly 6 may be in butt joint with the excreta collection device 5 backwards. The left buckling member 50 may be buckled on the second left buckling portion 700, and the right buckling member 51 may be buckled on the second right buckling portion 710. When the cleaning assembly 6 is in butt joint with the excreta collection device 5, the left buckling protrusion 500 snaps with the second left buckling portion 700, and the right buckling protrusion 510 snaps with the second right buckling portion 710. Along with the forward insertion of the front end of the excreta collection device 5 into the second slot 80, the left buckling member 50 snaps with the second left buckling portion 700, and the right buckling member 51 snaps with the second right buckling portion 710. Thus, the excreta collection device 5 and the cleaning assembly 6 are locked together, and rapid and convenient butt joint between the cleaning assembly 6 and the excreta collection device 5 is achieved.

When the cleaning assembly 6 is in butt joint with the excreta collection device 5, the frame opening portion 7 abuts on the excreta collection device 5. When the cleaning assembly 6 is in butt joint with the excreta collection device 5, the frame opening portion 7 abuts on the body portion 53 and the tray portion 52. The excreta collection device 5 may be in butt joint with the cleaning assembly 6 forwards. After the excreta collection device 5 is in butt joint with the cleaning assembly 6 forwards, the cleaning assembly 6 is located at the front side of the body portion 53. After the cleaning assembly 6 is in butt joint with the excreta collection device 5 backwards, the second left connection portion 70 and the second right connection portion 71 are located at the front side of the body portion 53, and abut against the body portion 53 separately. After the cleaning assembly 6 is in butt joint with the excreta collection device 5 backwards, the third U-shaped portion 72 is located at the upper side of the tray portion 52, and abuts on the tray portion 52. After the cleaning assembly 6 is in butt joint with the excreta collection device 5 backwards, the fourth U-shaped portion 73 is located at the front side of the body portion 53, and abuts on the body portion 53.

When the cleaning assembly 6 is in butt joint with the excreta collection device 5, the left buckling member 50 may keep in a state of snapping with the second left buckling portion 700 under the action of an elastic force of the left elastic member 540, and the right buckling member 51 may keep in a state of snapping with the second right buckling portion 710 under the action of an elastic force of the right elastic member 541. While the cleaning assembly 6 is in butt joint with the excreta collection device 5 backwards, the left buckling member 50 snaps with the second left buckling portion 700 under the action of the elastic force of the left elastic member 540, and the right buckling member 51 snaps with the second right buckling portion 710 under the action of the elastic force of the right elastic member 541. **In a** process that the cleaning assembly 6 is in butt joint with the excreta collection device 5 backwards, the left buckling protrusion 500 is buckled rightwards on the second left buckling portion 700 through elastic pushing of the left elastic member 540 on the left buckling member 50, and the right buckling protrusion 510 is buckled leftwards on the second right buckling portion 710 through elastic pushing of the right elastic member 541 on the right buckling member 51. Thus, the left buckling member 50 and the right buckling member 51 are basically synchronously and automatically buckled on the corresponding left buckling portion 400 and right buckling portion 410 through elastic pushing by the left elastic member 540 and the right elastic member 541.

The second left connection portion 70 is further provided with a third left dented region 701, and the second right connection portion 71 is further provided with a third right dented region 711. The second left buckling portion 700 is arranged in the third left dented region 701, and the second right buckling portion 710 is arranged in the third right dented region 711. When the cleaning assembly 6 is in butt joint with the excreta collection device 5, the front portion of the left buckling member 50 is embedded in the third left dented region 701, and the front portion of the right buckling member 51 is embedded in the third right dented region 711. The left buckling protrusion 500 is located at the front right side of the left buckling member 50, and the right buckling protrusion 510 is located on the front left side of the right buckling member 51. During unlocking, the rear portion of the left buckling member 50 and the rear portion of the right buckling member 51 are pressed in the direction close to each other with hands, so that the front portion of the left buckling member 50 tilts leftwards, and the front portion of the right buckling member 51 tilts rightwards. Thus, the left buckling protrusion 500 moves leftwards to be separated from the second left buckling portion 700 to release buckling, and the right buckling protrusion 510 moves rightwards to be separated from the second right buckling portion 710 to release buckling. To be specific, the excreta collection device 5 may be separated from the cleaning assembly 6 backwards in the front-rear direction.

The handle 86 may be hold with hands to be configured to move the cleaning assembly 6. The handle 86 may be hold with hands, so that the cleaning assembly 6 is in butt joint with the excreta collection device 5 backwards. The handle 86 is located at one side, facing away from the excreta collection device 5, of the cleaning assembly 6. The mounting hole 85 is located at one end of the handle 86. The handle 86 is arch-shaped, and is connected between the third U-shaped portion 72 and the fourth U-shaped portion 73. The handle 86 is connected between the upper side of the third U-shaped portion 72 and the front side of the fourth U-shaped portion 73. The handle 86 extends between a rear upper end of the cleaning assembly 6 and a front lower end of the cleaning assembly 6, and is located at one side, facing away from the excreta collection device 5, of the outer wall 820. A rear upper end of the handle 86 and a front lower end of the handle 86 are integrally connected to the outer wall 820 separately. The outer wall 820 is configured to delimit the concave space 82, and the frame opening portion 7 forms an annular edge of the outer wall 820. The rear upper end of the handle 86 and the front lower end of the handle 86 form two opposite ends of the handle 86, the mounting hole 85 is adjacent to the front lower end of the handle 86, and the mounting hole 85 is located at the upper side of the third U-shaped portion 72.

When the cleaning assembly 6 is in butt joint with the excreta collection device 5, the air intake check valve 83 is adjacent to the first spray nozzle 560, and is located at the upper side of the first spray nozzle 560. The protective plate 84 is located in the mounting hole 85, an inner surface of the mounting hole 85 includes an abutment surface 850, the protective plate 84 abuts on the abutment surface 850. The abutment surface 850 is annular, and the protective plate 84 abuts on the abutment surface 850 forwards. The air intake check valve 83 is located in the mounting hole 85, and interferes with an inner peripheral surface of the mounting hole 85 to fix the position relative to the mounting hole 85. The protective plate 84 is sandwiched between the air intake check valve 83 and the abutment surface 850. The air intake check valve 83 includes a valve core 830, a front cover 831, a rear cover 832, a first sealing ring 833, a second sealing ring 834, a first spring 835, and a flexible shock absorber 836. The front cover 831 is in butt joint with the rear cover 832, and a buckling mechanism (for example, a buckling protrusion and a snap groove) is arranged at a position for the butt joint between the front cover 831 and the rear cover 832. The front cover 831 is in snap fit with the rear cover 832 through the above buckling mechanism. The first sealing ring 833 is located at a position where the front cover 831 is in butt joint with the rear cover 832, and is adjacent to the above buckling mechanism. The first sealing ring 833 is clamped and fixed by the front cover 831 and the rear cover 832. The valve core 830 includes a valve rod 8300 and a valve cap 8301, where the first spring 835 sleeves the valve rod 8300, the first spring 835 is located between the valve cap 8301 and the rear cover 832, and the first spring 835 is in a compressed state between the valve cap 8301 and the rear cover 832. The air intake check valve 83 is normally closed. The valve cap 8301 abuts on the first sealing ring 833 under pushing by the first spring 835, so that the air intake check valve 83 is closed. The second sealing ring 834 sleeves the front cover 831, and is attached to an inner peripheral surface of the mounting hole 85, to seal between the inner peripheral surface of the mounting hole 85 and the air intake check valve 83. The flexible shock absorber 836 is mounted on the valve rod 8300, and takes a role of shock absorption between the valve rod 8300 and the rear cover 832, thereby reducing noise. The rear cover 832 is provided with a penetrated-through hole 8320 corresponding to the valve rod 8300, and the flexible shock absorber 836 takes a role of shock absorption between the valve rod 8300 and an inner surface of the penetrated-through hole 8320 to suppress direct collision between the valve rod 8300 and the rear cover 832, thereby reducing noise. If no flexible shock absorber 836 is provided, when the cleaning assembly 6 is in butt joint with the excreta collection device 5 and the air intake check valve 83 is opened under the suction of the negative pressure source 100, the valve core 830 and the valve rod 8300 thereof vibrate under the action of airflow, so that the noise of collision with the rear cover 832 is likely to be generated. In the example, the flexible shock absorber 836 is clamped on the valve rod 8300.

When the cleaning assembly 6 is in butt joint with the excreta collection device 5, an inner chamber 90 is formed between the cleaning assembly 6 and the excreta collection device 5. The excreta recess 520 forms a part of the inner chamber 90. When the cleaning assembly 6 is in butt joint with the excreta collection device 5, the concave space 82 is combined with the excreta recess 520 to form the inner chamber 90, and the concave space 82 forms a part of the inner chamber. The first spray nozzle 270 may spray water into the inner chamber 90 to fill the inner chamber 90 with water (if the water contains a disinfectant material, a disinfectant solution is formed). The first spray nozzle 560 may supply water to the excreta suction pipeline 11 via the inner chamber 90, and the inner chamber 90, the excreta discharge pipeline 534, and the first excreta suction hose device 110 may be in fluid communication in sequence. Further, the inner chamber 90, the excreta discharge pipeline 534, the first excreta suction hose device 110, the second excreta suction hose device, and the excreta container 103 may be in fluid communication in sequence. When the cleaning assembly 6 is in butt joint with the excreta collection device 5, the first spray nozzle 560 is exposed to an interior of the inner chamber 90 to spray water into the inner chamber 90.

When the cleaning assembly 6 is in butt joint with the excreta collection device 5, the flexible sealing gasket 580 is pressed between the excreta collection device 5 and the frame opening portion 7 to seal between the excreta collection device 5 and the frame opening portion 7, so that the inner chamber 90 is isolated from the external environment, and the liquid in the inner chamber 90 is prevented from leaking out from between the excreta collection device 5 and the frame opening portion 7. The flexible sealing gasket 580 is annular in shape corresponding to the frame opening portion 7. The frame opening portion 7 is further provided with a second annular abutting surface 74 corresponding to the flexible sealing gasket 580, where the second annular abutting surface 74 is arranged on the second left connection portion 70, the second right connection portion 71, the third U-shaped portion 72, and the fourth U-shaped portion 73. When the cleaning assembly 6 is in butt joint with the excreta collection device 5, the flexible sealing gasket 580 is pressed against the second annular abutting surface 74 to seal between the excreta collection device 5 and the frame opening portion 7.

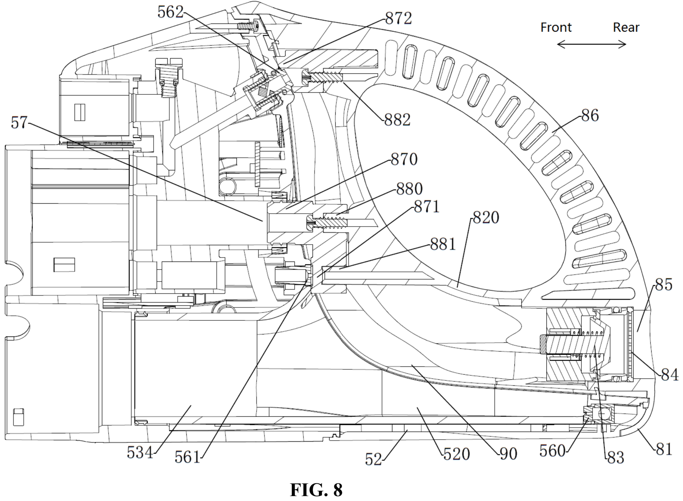

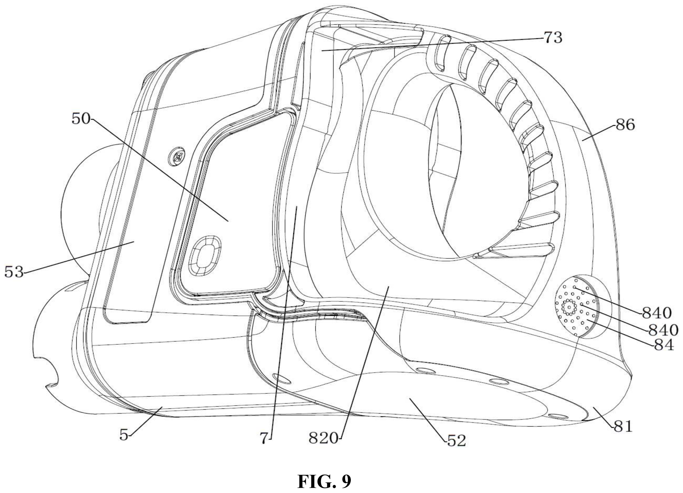

With reference to Figs. 8 and 9 emphatically, when in butt joint with the excreta collection device 5, the cleaning assembly 6 covers the excreta recess 520. From another view, when the excreta collection device 5 is in butt joint with the cleaning assembly 6, the cleaning assembly 6 covers the excreta recess 520. When in butt joint with the excreta collection device 5 backwards, the cleaning assembly 6 covers the front side of the body portion 53 and the upper side of the tray portion 52. When the cleaning assembly 6 is in butt joint with the excreta collection device 5, the first plug 870 blocks the drying air outlet 57 to isolate the drying air outlet 57 from the inner chamber 90 , to prevent liquid in the inner chamber 90 from entering the drying air outlet 57, and thus the cleaning and disinfecting liquid is prevented from entering or polluting the drying air outlet 57. When the cleaning assembly 6 is in butt joint with the excreta collection device 5, the second plug 871 blocks the second spray nozzle 561 to isolate the second spray nozzle 561 from the inner chamber 90 , to prevent liquid in the inner chamber 90 from entering the second spray nozzle 561, so that the cleaning and disinfecting liquid is prevented from entering or polluting the second spray nozzle 561. When the cleaning assembly 6 is in butt joint with the excreta collection device 5, the third plug 872 blocks the third spray nozzle 562 to isolate the third spray nozzle 562 from the inner chamber 90 , to prevent liquid in the inner chamber 90 from entering the third spray nozzle 562, so that the cleaning and disinfecting liquid is prevented from entering or polluting the third spray nozzle 562. The first plug 870 is made of a flexible material, such as rubber. The cleaning assembly 6 is further provided with a first location protrusion 880, where the first location protrusion 880 is integrally connected to the outer wall 820, and located in the concave space 82. The first location protrusion 880 is configured to locate the first plug 870, and the first plug 870 sleeves the first location protrusion 880, and is locked on the first location protrusion 880 through screws. Further, the front end of the first plug 870 sleeves the first location protrusion 880. When the cleaning assembly 6 is in butt joint with the excreta collection device 5, the first plug 870 is capable of inserting into the drying air outlet 57 to block the drying air outlet 57. Further, after the cleaning assembly 6 is in butt joint with the excreta collection device 5 backwards, the rear end of the first plug 870 is capable of inserting into the drying air outlet 57 to block the drying air outlet 57.

The second plug 871 is made of a flexible material, such as rubber. The second plug 871 is located in the concave space 82. The cleaning assembly 6 is further provided with a second location protrusion 881, where the second location protrusion 881 is integrally connected to the outer wall 820, located in the concave space 82, to locate the second plug 871. The second plug 871 sleeves the second location protrusion 881. Specifically, a front end of the second plug 871 sleeves the second location protrusion 881. The second plug 871 and the first plug 870 are arranged in parallel. Correspondingly, the second location protrusion 881 and the first location protrusion 880 are arranged in parallel, and the second plug 871 is integrally connected to the first plug 870, so that the fixation of the first plug 870 assists with the fixation of the second plug 871. After the cleaning assembly 6 is in butt joint with the excreta collection device 5 backwards, the second plug 871 abuts on the second spray nozzle 561 to block the second spray nozzle 561. Further, after the cleaning assembly 6 is in butt joint with the excreta collection device 5 backwards, a rear end of the second plug 871 abuts on the second spray nozzle 561 backwards to block the second spray nozzle 561.

The third plug 872 is made of a flexible material, such as rubber. The third plug 872 is located in the concave space 82. The cleaning assembly 6 is further provided with a third location protrusion 882, where the third location protrusion 882 is integrally connected to the outer wall 820, located in the concave space 82, to locate the third plug 872. The third plug 872 sleeves the third location protrusion 882. Specifically, a front end of the third plug 872 sleeves the third location protrusion 882. When the cleaning assembly 6 is in butt joint with the excreta collection device 5, one end of the third plug 872 covers the third spray nozzle 562 to block the second spray nozzle 561. Further, when the cleaning assembly 6 is in butt joint with the excreta collection device 5 backwards, the rear end of the third plug 872 covers the third spray nozzle 562 to block the third spray nozzle 562. When the cleaning assembly 6 is in butt joint with the excreta collection device 5, the first location protrusion 880, the second location protrusion 881, and the third location protrusion 882 are located between the outer wall 820 and the excreta collection device 5 separately.

The second plug 871 and the third plug 872 are located in the inner chamber 90. The air intake check valve 83 is configured for communication between the inner chamber 90 and the external environment, and configured for unidirectional air intake into the inner chamber 90. After the air intake check valve 83 is opened, air from the external environment may enter the inner chamber 90 via the air intake check valve 83. Under the suction of the negative pressure source 100, the air intake check valve 83 is opened, so that the air from the external environment may enter the inner chamber 90 via the air intake check valve 83. When the cleaning assembly 6 is in butt joint with the excreta collection device 5, the excreta discharge pipeline 534 is in fluid communication with the inner chamber 90, the first spray nozzle 270 is capable of spraying water into the inner chamber 90, and the water in the inner chamber 90 may be discharged via the excreta discharge pipeline 534. Specifically, the water in the inner chamber 90 may gradually flow into the first excreta suction hose device 110 via the excreta discharge pipeline 534. When the cleaning assembly 6 is in butt joint with the excreta collection device 5, under the suction of the negative pressure source 100, the air inlet check valve 83 is opened, and external air enters the inner chamber 90 via the air inlet check valve 83. Specifically, when the cleaning assembly 6 is in butt joint with the excreta collection device 5, under the suction of the negative pressure source 100, the air pressure in the inner chamber 90 is negative relative to the outside, so that the valve cap 8301 is pushed away from the first sealing ring 833 under the pressure of the external air, the air intake check valve 83 is opened, and the external air enters the inner chamber 90 via the air intake check valve 83. The outer wall 820 is integrally connected to the handle 86, and made of a plastic material. **In** the example, the outer wall 820 is transparent to observe a condition in the inner chamber 90. The condition in the inner chamber 90 may be observed through the outer wall 820. When the cleaning assembly 6 is in butt joint with the excreta collection device 5, the outer wall 820, the body portion 53, and the tray portion 52 jointly delimit the inner chamber 90. After the cleaning assembly 6 is in butt joint with the excreta collection device 5 backwards, the outer wall 820 covers the front side of the body portion 53 and the upper side of the tray portion 52, to delimit the inner chamber 90 along with the body portion 53 and the tray portion 52 jointly.

The first spray nozzle 560 may spray water into the inner chamber 90 to fill the inner chamber 90, the excreta discharge pipeline 534, and the first excreta suction hose device 110 with water (if the water contains a disinfectant material, a disinfectant solution is formed). Thus, the immersed disinfection of the excreta recess 520, etc. of the excreta collection device 5, and the interior of the excreta suction pipeline 11 are promoted without disassembling the excreta collection device 5, the excreta suction pipeline 11, and the main machine 10, and the cleaning and disinfecting are simpler and more convenient. After the cleaning assembly 6 is in butt joint with the excreta collection device 5, the cleaning assembly 6, the excreta collection device 5, and the first excreta suction hose device 110 may be placed on the ground, so that the first spray nozzle 270 continuously is capable of spraying water, and the sprayed water may fill the inner chamber 90, the excreta discharge pipeline 534, and the first excreta suction hose device 110. One end of the first excreta suction hose device 110 is connected to the excreta collection device 5, and the other end of the first excreta suction hose device 110 is connected to the main machine 10. The other end of the first excreta suction hose device 110 is higher in position. When water has overflowed the other end of the first excreta suction hose device 110, it indicates that the inner chamber 90, the excreta discharge pipeline 534, and the first excreta suction hose device 110 have been filled with water. The water sprayed out from the first spray nozzle 270 is fused with the disinfectant material to form a disinfectant solution for cleaning and disinfecting the interiors of the excreta recess 520, the excreta discharge pipeline 534, and the first excreta suction hose device 110. After the disinfectant solution basically fills the first excreta suction hose device 110, the water pump 107 may be shut down, so that the first spray nozzle 270 stops spraying water, and the disinfectant solution remains in the inner chamber 90, the excreta discharge pipeline 534, and the first excreta suction hose device 110 to perform immersed disinfection on the excreta recess 520, the excreta discharge pipeline 534, and the first excreta suction hose device 110. The immersed disinfection time may be set according to the specific demand for sterilization and disinfection. After the immersed disinfection is completed, the disinfectant solution in the excreta recess 520, the excreta discharge pipeline 534, and the first excreta suction hose device 110 is sucked into the excreta container 103 under the suction of the negative pressure source 100. The air intake check valve 83 is opened under the suction of the negative pressure source 100, so that the disinfectant solution in the excreta recess 520, the excreta discharge pipeline 534, and the first excreta suction hose device 110 may be smoothly and completely sucked. The disinfectant material may be powdery, granular, liquid, etc. Citric acid, dibromohydantoin, effervescent tablets, a chlorine dioxide disinfectant agent/liquid, bromine-containing disinfectant agent, etc. may be selected as the disinfectant material.

When immersed disinfection is required, the excreta collection device 5 releases the butt joint with the connection bracket 4 firstly, so that the briefs device 2 may be separated from the excreta collection device 5 to remove the briefs device 2. The disinfectant material is put into the excreta collection device 5 or the first excreta suction hose device 110. The disinfectant material is preferentially put into the excreta recess 520, which is convenient. The disinfectant material may alternatively be put into the excreta discharge pipeline 534. The cleaning assembly 6 is in butt joint with the excreta collection device 5, so that the concave space 82 and the excreta recess 520 form the inner chamber 90. The flexible sealing gasket 580 takes a role of sealing between the excreta collection device 5 and the cleaning assembly 6 to prevent the liquid leakage from the inner chamber 90. The water pump 107 is activated, so that the first spray nozzle 270 is capable of spraying water, and the water sprayed out from the first spray nozzle 270 is fused with the disinfectant material to form the disinfectant solution. The disinfectant solution remains in the inner chamber 90, the excreta discharge pipeline 534, and the first excreta suction hose device 110 for preset immersed disinfection time, to perform immersed disinfection on the interiors of the excreta recess 520, the excreta discharge pipeline 534, and the first excreta suction hose device 110. The preset immersed disinfection time may be set according to the specific requirements for sterilization and disinfection, and is generally 21 to 60 minutes. After the immersed disinfection is completed, the disinfectant solution in the inner chamber 90, the excreta discharge pipeline 534, and the first excreta suction hose device 110 is sucked into the excreta container 103 under the suction of the negative pressure source 100. Then, the water pump 107 may be activated, the first spray nozzle 270 is capable of spraying water, and the negative pressure source 100 performs suction, so that the interiors of the inner chamber 90, the excreta discharge pipeline 534, and the first excreta suction hose device 110 are cleaned to remove a residual disinfectant solution. For the nursing machine, with the aid of the cleaning assembly 6, the immersed disinfection may be performed on the excreta recess 520, etc. of the excreta collection device 5, and the interior of the excreta suction pipeline 11 without disassembling the excreta collection device 5, the excreta suction pipeline 11, and the main machine 10, so that the cleaning and disinfecting are simpler and more convenient. The water spraying time of the first spray nozzle 270 may be selected according to the capacities of the inner chamber 90, the excreta discharge pipeline 534, and the first excreta suction hose device 110, and the water spraying amount of the first spray nozzle 270 per unit time. Thus, waste and an insufficient concentration of the disinfectant solution caused by overflow of some or most disinfectant solution from the first excreta suction hose device 110 and inflow into the excreta container 103 via the second excreta suction hose device because the first spray nozzle 270 sprays too much water for a too long time is avoided. If the interiors of the second excreta suction hose device and the excreta container 103 are to undergo immersed disinfection, the first spray nozzle 560 is capable of spraying water for a longer time to provide more disinfectant solution. The water in the clean water container 105 may also contain a disinfectant material, i.e. a disinfectant solution. Further, under driving by the water pump 107, the water containing the disinfectant material, i.e. the disinfectant solution, is directly sprayed out via the first spray nozzle 270. The disinfectant solution may be prepared in advance, so that the concentration is more accurate and controllable.

## Claims

1. A nursing machine, comprising an excreta collection device, wherein the excreta collection device is wearable on a body of a user to receive excreta of the user, and the excreta collection device comprises an excreta recess configured to receive the excreta of the user, and a first spray nozzle configured to spray water; the nursing machine further comprises a cleaning assembly, wherein when in butt joint with the excreta collection device, the cleaning assembly covers the excreta recess and the first spray nozzle; when the cleaning assembly is in butt joint with the excreta collection device, an inner chamber is formed between the cleaning assembly and the excreta collection device, and the excreta recess forms a part of the inner chamber; and the first spray nozzle is capable of spraying water into the inner chamber.

2. The nursing machine according to claim 1, wherein the cleaning assembly comprises a concave space, when the cleaning assembly is in butt joint with the excreta collection device, the concave space is combined with the excreta recess, and the concave space forms a part of the inner chamber.

3. The nursing machine according to claim 2, wherein the nursing machine further comprises a briefs device wearable on the body of the user, wherein the briefs device comprises a connection bracket and a crotch through opening, at least a part of the crotch through opening is delimited by the connection bracket, and after the briefs device is worn on the body of the user, the excreta collection device is capable of indirectly worn on the body of the user by being in butt joint with the connection bracket; the excreta collection device is configured for being optionally in butt joint with either one of the cleaning assembly and the connection bracket of the briefs device.

4. The nursing machine according to claim 3, wherein the cleaning assembly further comprises a frame opening portion, and the concave space is formed by recessing into the cleaning assembly from the frame opening portion; the excreta collection device is capable of being in butt joint with the frame opening portion detachably, and capable of being in butt joint with the connection bracket detachably; when the cleaning assembly is in butt joint with the excreta collection device, the frame opening portion abuts on the excreta collection device; and when the connection bracket of the briefs device is in butt joint with the excreta collection device, the connection bracket abuts on the excreta collection device.

5. The nursing machine according to claim 4, wherein the connection bracket comprises a first left connection portion and a first right connection portion, and the first left connection portion and the first right connection portion are located at a left side and a right side of the crotch through opening respectively; the first left connection portion comprises a first left buckling portion, and the first right connection portion comprises a first right buckling portion; the frame opening portion is annular, and the frame opening portion comprises a second left connection portion and a second right connection portion, wherein the second left connection portion and the second right connection portion are located at a left side and a right side of the concave space respectively; the second left connection portion comprises a second left buckling portion, the second right connection portion comprises a second right buckling portion, and the second left buckling portion and the second right buckling portion are located at a left side and a right side of the cleaning assembly respectively; the excreta collection device further comprises a locking mechanism, wherein the locking mechanism comprises a left buckling member and a right buckling member, and the left buckling member and the right buckling member are located at a left side and a right side of the excreta collection device respectively; the excreta collection device is further provided with a left elastic member corresponding to the left buckling member, and a right elastic member corresponding to the right buckling member; when the connection bracket of the briefs device is in butt joint with the excreta collection device, the left buckling member keeps in a state of snapping with the first left buckling portion under the action of an elastic force of the left elastic member, and the right buckling member keeps in a state of snapping with the first right buckling portion under the action of an elastic force of the right elastic member; and when the cleaning assembly is in butt joint with the excreta collection device, the left buckling member keeps in a state of snapping with the second left buckling portion under the action of an elastic force of the left elastic member, and the right buckling member keeps in a state of snapping with the second right buckling portion under the action of an elastic force of the right elastic member.

6. The nursing machine according to claim 5, wherein the frame opening portion further comprises a third U-shaped portion and a fourth U-shaped portion, the third U-shaped portion is located at a front lower portion of the frame opening portion, and the fourth U-shaped portion is located at a rear upper portion of the frame opening portion; the third U-shaped portion, the second left connection portion, the fourth U-shaped portion, and the second right connection portion are connected in sequence to form a loop; the connection bracket is further provided with a first slot; the cleaning assembly is further provided with a second slot and a second cambered stop wall, wherein the second slot is located at a front bottom side of the cleaning assembly, and a front end of the excreta collection device is capable of inserting into the second slot forwards; the second cambered stop wall is configured to delimit the second slot, the second cambered stop wall is connected to the third U-shaped portion, and located at a lower side of the third U-shaped portion, the second slot is located at the lower side of the third U-shaped portion, and the second slot is formed between the third U-shaped portion and the second cambered stop wall; when the connection bracket of the briefs device is in butt joint with the excreta collection device, the front end of the excreta collection device is embedded in the first slot; when the cleaning assembly is in butt joint with the excreta collection device, the front end of the excreta collection device is embedded in the second slot; and along with forward insertion of the front end of the excreta collection device into the second slot, the left buckling member snaps with the second left buckling portion, and the right buckling member snaps with the second right buckling portion.

7. The nursing machine according to claim 5, wherein the frame opening portion comprises a third U-shaped portion and a fourth U-shaped portion, the third U-shaped portion, the second left connection portion, the fourth U-shaped portion, and the second right connection portion are connected in sequence to form a loop; the excreta collection device further comprises a tray portion and a body portion; the left elastic member is located between the body portion and the left buckling member, and the right elastic member is located between the body portion and the right buckling member; the tray portion extends forwards from a lower portion of the body portion, and the excreta recess is concavely arranged at the tray portion; the left buckling member and the right buckling member are pivotally connected to the body portion, and the left buckling member and the right buckling member are located at a left side and a right side of the body portion respectively; when the connection bracket of the briefs device is in butt joint with the excreta collection device, the connection bracket abuts on the body portion and the tray portion; when the cleaning assembly is in butt joint with the excreta collection device, the frame opening portion abuts on the body portion and the tray portion; after in butt joint with the excreta collection device backwards, the cleaning assembly is located at a front side of the body portion; after the cleaning assembly is in butt joint with the excreta collection device backwards, the second left connection portion and the second right connection portion are located at the front side of the body portion and abut against the body portion separately; after the cleaning assembly is in butt joint with the excreta collection device backwards, the third U-shaped portion is located at an upper side of the tray portion and abuts on to the tray portion; and after the cleaning assembly is in butt joint with the excreta collection device backwards, the fourth U-shaped portion is located at the front side of the body portion and abuts on the body portion.

8. The nursing machine according to claim 4, wherein the excreta collection device further comprises a flexible sealing gasket, and the flexible sealing gasket surrounds an outer periphery of the excreta recess; when the connection bracket of the briefs device is in butt joint with the excreta collection device, the flexible sealing gasket is pressed between the excreta collection device and the connection bracket, to seal between the excreta collection device and the connection bracket; and when the cleaning assembly is in butt joint with the excreta collection device, the flexible sealing gasket is configured to seal between the excreta collection device and the frame opening portion, to isolate the inner chamber from an external environment.

9. The nursing machine according to claim 8, wherein the cleaning assembly further comprises an outer wall configured to delimit the concave space, and the frame opening portion forms an annular edge of the outer wall; the excreta collection device further comprises an annular groove, the flexible sealing gasket is annular in shape corresponding to the annular groove, the flexible sealing gasket is arranged in the annular groove, a part of the annular groove is dented in a front side of a body portion, and the other part of the annular groove is dented in an upper side of a tray portion; the flexible sealing gasket and the frame opening portion are annular in shape corresponding to each other; the frame opening portion is further provided with a second annular abutting surface corresponding to the flexible sealing gasket; and when the cleaning assembly is in butt joint with the excreta collection device, the flexible sealing gasket is pressed against the second annular abutting surface.

10. The nursing machine according to claim 2, wherein the excreta collection device further comprises a tray portion and a body portion; the tray portion extends forwards from a lower portion of the body portion, and the excreta recess is concavely arranged at the tray portion; the cleaning assembly comprises an outer wall configured to delimit a concave space; and after the cleaning assembly is in butt joint with the excreta collection device backwards, the outer wall covers a front side of the body portion and an upper side of the tray portion, to delimit the inner chamber along with the body portion and the tray portion jointly.

11. The nursing machine according to claim 10, wherein the outer wall is transparent, to observe a condition in the inner chamber through the outer wall; and the cleaning assembly comprises a handle, a mounting hole, and an air intake check valve, wherein the handle is located on one side, facing away from the excreta collection device, of the outer wall, the air intake check valve is located in the mounting hole, and the air intake check valve is configured for unidirectional air intake into the concave space.

12. The nursing machine according to claim 1, wherein the nursing machine further comprises a briefs device wearable on the body of the user, wherein the briefs device comprises a connection bracket, the connection bracket is located at a crotch portion of the briefs device, the excreta collection device is capable of releasing butt joint with the connection bracket to butt with the cleaning assembly, and capable of releasing butt joint with the cleaning assembly to butt with the connection bracket; the excreta collection device comprises a left buckling member and a right buckling member; when the excreta collection device is in butt joint with the connection bracket, the left buckling member and the right buckling member snap with the connection bracket; and when the excreta collection device is in butt joint with the cleaning assembly, the left buckling member and the right buckling member snap with the cleaning assembly.

13. The nursing machine according to claim 1, wherein the excreta collection device further comprises a tray portion and a body portion; the tray portion extends forwards from a lower portion of the body portion, and the excreta recess is concavely arranged at the tray portion; and after in butt joint with the excreta collection device backwards, the cleaning assembly covers a front side of the body portion and an upper side of the tray portion.

14. The nursing machine according to claim 13, wherein the excreta collection device further comprises a second spray nozzle configured to spray water, and a drying air outlet, the first spray nozzle is arranged at the tray portion, the first spray nozzle is located at a front side of the excreta recess, and the first spray nozzle flushes away excreta in the excreta recess; the second spray nozzle is arranged at the body portion, the second spray nozzle is located at the front side of the body portion, the second spray nozzle is located at a rear upper side of the excreta recess, and the second spray nozzle is configured to spray water forwards to flush an anus of the body of the user; the cleaning assembly comprises a first plug and a second plug; when the cleaning assembly is in butt joint with the excreta collection device, the first plug blocks the drying air outlet to isolate the drying air outlet from the inner chamber, to prevent liquid in the inner chamber from entering the drying air outlet; and when the cleaning assembly is in butt joint with the excreta collection device, the second plug blocks the second spray nozzle to isolate the second spray nozzle from the inner chamber , to prevent liquid in the inner chamber from entering the second spray nozzle.

15. The nursing machine according to claim 14, wherein the excreta collection device further comprises a urine cover and a third spray nozzle configured to spray water, the third spray nozzle is arranged at the body portion, the third spray nozzle is located at the front side of the body portion, the third spray nozzle is located at an upper side of the second spray nozzle, and the third spray nozzle is configured to flush an interior of the urine cover and/or a urination portion of the body of the user; the drying air outlet is arranged at the body portion, the drying air outlet is located at the upper side of the second spray nozzle, and the drying air outlet is located between the second spray nozzle and the third spray nozzle; the cleaning assembly comprises a third plug; when the cleaning assembly is in butt joint with the excreta collection device, the third plug blocks the third spray nozzle to isolate the third spray nozzle from the inner chamber, to prevent liquid in the inner chamber from entering the third spray nozzle.

16. The nursing machine according to claim 15, wherein the second plug and the third plug are located in the inner chamber; the cleaning assembly further comprises an outer wall configured to delimit a concave space, a first location protrusion, a second location protrusion, and a third location protrusion; when the cleaning assembly is in butt joint with the excreta collection device, the first location protrusion, the second location protrusion, and the third location protrusion are located between the outer wall and the excreta collection device separately; the first location protrusion, the second location protrusion, and the third location protrusion are integrally connected to the outer wall separately; the first location protrusion is located in the concave space, and the first location protrusion is configured to locate the first plug; the second location protrusion is located in the concave space, and the second location protrusion is configured to locate the second plug; the third location protrusion is located in the concave space, and the third location protrusion is configured to locate the third plug; when the cleaning assembly is in butt joint with the excreta collection device, the first plug is inserted into the drying air outlet to block the drying air outlet; and after the cleaning assembly is in butt joint with the excreta collection device backwards, a rear end of the first plug is inserted into the drying air outlet.

17. The nursing machine according to claim 1, wherein when the cleaning assembly is in butt joint with the excreta collection device, the first spray nozzle is exposed to an interior of the inner chamber, and the first spray nozzle is capable of spraying water into the inner chamber to fill the inner chamber with water.

18. The nursing machine according to claim 17, wherein the excreta collection device further comprises an excreta discharge pipeline, a front end of the excreta discharge pipeline is located at a rear end of the excreta recess, and directly communicates with the excreta recess; the nursing machine further comprises a main machine and an excreta suction pipeline, wherein the excreta discharge pipeline forms a part of the excreta suction pipeline; the excreta suction pipeline further comprises a first excreta suction hose device, wherein one end of the first excreta suction hose device is in butt joint with the excreta collection device, and an opposite end of the first excreta suction hose device is in butt joint with the main machine; the inner chamber, the excreta discharge pipeline, and the first excreta suction hose device are in fluid communication in sequence; the first spray nozzle supplies water to the excreta suction pipeline via the inner chamber, and the first spray nozzle is capable of spraying water into the inner chamber to fill the inner chamber, the excreta discharge pipeline, and the first excreta suction hose device with water; a first water conveying pipeline is configured to supply water to the first spray nozzle; and the main machine comprises a water pump, wherein the water pump drives water to be conveyed via the first water conveying pipeline and sprayed out via the first spray nozzle.

19. The nursing machine according to claim 1, wherein the nursing machine further comprises a main machine and an excreta suction pipeline; wherein the excreta collection device further comprises an excreta discharge pipeline, the excreta discharge pipeline forms a part of the excreta suction pipeline, the excreta suction pipeline further comprises a first excreta suction hose device, and the first excreta suction hose device is configured for fluid communication between the excreta collection device and the main machine; the main machine comprises a negative pressure source providing power for sucking excreta, and an excreta container, wherein under the suction of the negative pressure source, the excreta in the excreta recess are sucked into the excreta container via the excreta suction pipeline; the inner chamber, the excreta discharge pipeline, and the first excreta suction hose device are in fluid communication in sequence, and the first spray nozzle supplies water to the excreta suction pipeline via the inner chamber; the cleaning assembly further comprises an air intake check valve, wherein the air intake check valve is configured for unidirectional air intake into the inner chamber, and after the air intake check valve is opened, air in an external environment enters the inner chamber via the air intake check valve; and under the suction of the negative pressure source, the air intake check valve is opened, and the air in the external environment enters the inner chamber via the air intake check valve.

20. A cleaning assembly for a nursing machine, wherein the nursing machine comprises an excreta collection device, the excreta collection device is wearable on a body of a user to receive excreta of the user; the excreta collection device comprises an excreta recess configured to receive the excreta of the user, and a first spray nozzle configured to spray water; when in butt joint with the excreta collection device, the cleaning assembly covers the excreta recess and the first spray nozzle; and when the cleaning assembly is in butt joint with the excreta collection device, an inner chamber is formed between the cleaning assembly and the excreta collection device, the excreta recess forms a part of the inner chamber, and the first spray nozzle is capable of spraying water into the inner chamber.
